# EUROPEAN PATENT APPLICATION

(11) **EP 2 070 908 A1**
(43) Date of publication of application: **17.06.2009**
(21) Application number: 07828202.7
(22) Date of filing: 07.09.2007
(51) Int. Cl.: C07C 233/80, A61K 31/167, A61K 31/44, A61P 7/02, A61P 25/04, A61P 29/00, A61P 35/00, A61P 43/00, C07C 237/34, C07D 213/75

(54) **COMPOUND HAVING BENZAMIDE SKELETON AND CYCLOOXYGENASE (COX-1)-SELECTIVE INHIBITORY ACTIVITY**

(30) Priority: 07.09.2006 JP 2006242861
(71) Applicant: National University Corporation Okayama University, Okayama-shi, Okayama 700-8530 (JP)
(72) Inventor: KAKUTA, Hiroki, Okayama-shi Okayama 700-8530 (JP); SASAKI, Kenji, Okayama-shi Okayama 700-8530 (JP); ODA, Hiroyuki, Okayama-shi Okayama 700-8530 (JP); ZHENG, Xiaoxia, Okayama-shi Okayama 700-8530 (JP); HARADA, Shun, Okayama-shi Okayama 700-8530 (JP)
(74) Representative: Woods, Geoffrey Corlett
(86) International application number: PCT/JP2007/067493
(87) International publication number: WO 2008/029912

(57) **Abstract**

This invention provides a novel COX-1-selective inhibitor. This invention relates to a novel compound represented by the formula below or a salt thereof. This invention also relates to an analgesic agent, an antiinflammatory agent, an antitumor agent, an antiplatelet aggregation agent, and a cyclooxygenase-1-selective inhibitor comprising, as an active ingredient, such compound or salt thereof.

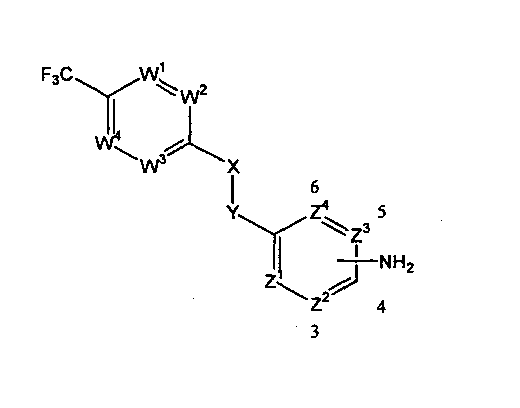

## Description

### Technical Field

The present invention relates to an analgesic agent, an antiinflammatory agent, an antitumor agent, an antiplatelet aggregation agent, and a cyclooxygenase-1 (COX-1)-selective inhibitor.

### Background Art

Cyclooxygenase-1 (COX-1)-selective inhibitors are known to exhibit analgesic activity or neovascularization inhibitory activity without causing gastrointestinal trouble, and aspirin having low COX-1 inhibitory activity is known to have antiplatelet aggregation activity and activity of lowering colon cancer mortality. COX-1-selective inhibitors have hardly been studied since inhibition of COX-1 was considered to cause gastrointestinal trouble induced by nonsteroidal anti-inflammatory drugs (NSAIDs), such as indomethacin. However, it has been reported that such gastrointestinal trouble is observed when both COX-1 and COX-2 subtypes are inhibited, and no gastrointestinal trouble is observed when only one thereof is inhibited. Since the correlation of some COX-2-selective inhibitors (e.g., rofecoxib) with heart stroke was reported around 2003, research into COX-1 inhibitors has been gradually resumed.

An example of a COX-1-selective inhibitor with analgesic activity that has been used for clinical applications is 3,4-diaryl-isoxazole-5-acetic acid (tradename: Mofezolac) (see JP Patent Publication (kokai) No. S56-59764 A (1981)).

### Disclosure of the Invention

The present invention provides a novel COX-1-selective inhibitor.

Mofezolac is an acidic compound. If an active ingredient of a COX-1-selective inhibitor is a neutral or basic compound, the metabolic pathway of such compound can be different from that of Mofezolac. When drug hypersensitivity to an acidic COX-1 inhibitor, such as Mofezolac, is observed, such COX-1-selective inhibitor can serve as an alternative drug. Therefore, the present invention provides a COX-1-selective inhibitor comprising, as an active ingredient, a neutral or basic compound.

The present inventors have synthesized various types of benzanilide derivatives by mimicking the conformation of indomethacin in cyclooxygenase-1 (COX-1), which is known to be a strong cyclooxygenase inhibitor, and they have inspected the cyclooxygenase inhibitory activity. As a result, they found that amino-containing compounds have COX-1-selective inhibitory activity. Further, the compound of interest was converted into a pyridine ring- or pirimidine ring-containing compound. As a result, they found compounds exhibiting high water solubility and analgesic activity in mice.

The present invention includes the following inventions.
(1) A compound represented by a formula below or a salt thereof: 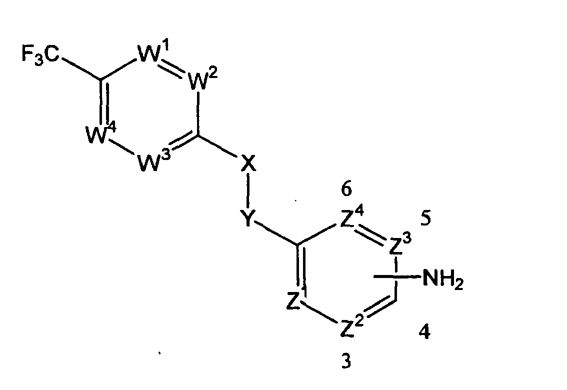 wherein either X or Y represents CO and the other represents NH; W¹, W², W³, and W⁴ each independently represent CH or N; Z¹, Z², Z³, and Z⁴ each independently represent CH or N (provided that Z², Z³, or Z⁴ to which an amino group binds is CH); and an amino group is located at position 3, 4, 5, or 6.
(2) The compound according to (1) or a salt thereof, wherein X represents CO; Y represents NH; Z¹ represents N; Z², Z³, and Z⁴ each independently represent CH; W¹, W², W³, and W⁴ each independently represent CH; and an amino group is located at position 4 (such compound being referred to as "Compound 1" herein).
(3) The compound according to (1) or a salt thereof, wherein X represents CO; Y represents NH; Z¹ represents N; Z², Z³, and Z⁴ each independently represent CH; W¹, W², W³, and W⁴ each independently represent CH; and an amino group is located at position 6 (such compound being referred to as "Compound 2" herein).
(4) The compound according to (1) or a salt thereof, wherein X represents CO; Y represents NH; Z¹, Z², Z³, and Z⁴ each independently represent CH; W¹, W², W³, and W⁴ each independently represent CH; and an amino group is located at position 4 (such compound being referred to as "Compound 3" herein).
(5) The compound according to (1) or a salt thereof, wherein X represents CO; Y represents NH; Z¹, Z², Z³, and Z⁴ each independently represent CH; W¹, W², W³, and W⁴ each independently represent CH; and an amino group is located at position 5 (such compound being referred to as "Compound 4" herein).
(6) The compound according to (1) or a salt thereof, wherein X represents NH; Y represents CO; Z¹, Z², Z³, and Z⁴ each independently represent CH; W¹, W², W³, and W⁴ each independently represent CH; and an amino group is located at position 4 (such compound being referred to as "Compound 5" herein).
(7) The compound according to (1) or a salt thereof, wherein X represents NH; Y represents CO; Z¹, Z², Z³, and Z⁴ each independently represent CH; W¹, W², W³, and W⁴ each independently represent CH; and an amino group is located at position 5 (such compound being referred to as "Compound 6" herein).
(8) The compound according to (1) or a salt thereof, wherein X represents CO; Y represents NH; Z¹ and Z⁴ each independently represent N; Z² and Z⁴ each independently represent CH; W¹, W², W³, and W⁴ each independently represent CH; and an amino group is located at position 4 (such compound being referred to as "Compound 7" herein).
(9) The compound according to (1) or a salt thereof, wherein X represents CO; Y represents NH; Z¹ represents N; Z², Z³, and Z⁴ each independently represent CH; W¹ represents N, W², W³, and W⁴ each independently represent CH; and an amino group is located at position 4 (such compound being referred to as "Compound 8" herein).
(10) The compound according to (1) or a salt thereof, wherein X represents CO; Y represents NH; Z¹ represents N; Z¹, Z³, and Z⁴ each independently represent CH; W¹ and W² each independently represent N; W³, and W⁴ each independently represent CH; and an amino group is located at position 4 (such compound being referred to as "Compound 9" herein).
(11) The compound according to (1) or a salt thereof, wherein X represents NH; Y represents CO; Z¹ represents N; Z², Z³, and Z⁴ each independently represent CH; W¹, W², W³, and W⁴ each independently represent CH; and an amino group is located at position 4 (such compound being referred to as "Compound 10" herein).
(12) The compound according to (1) or a salt thereof, wherein X represents NH; Y represents CO; Z¹ represents N; Z², Z³, and Z⁴ each independently represent CH; W¹ represents N; W², W³, and W⁴ each independently represent CH; and an amino group is located at position 4 (such compound being referred to as "Compound 11" herein).
(13) The compound according to claim 1 or a salt thereof, wherein X represents NH; Y represents CO; Z¹ represents N; Z², Z³, and Z⁴ each independently represent CH; W² represents N; W¹, W³, and W⁴ each independently represent CH; and an amino group is located at position 4 (such compound being referred to as "Compound 12" herein).
(14) The compound according to claim 1 or a salt thereof, wherein X represents CO; Y represents NH; Z¹ represents N; Z², Z³, and Z⁴ each independently represent CH; W¹, W², W³, and W⁴ each independently represent CH; and an amino group is located at position 5 (such compound being referred to as "Compound 13" herein).
(15) The compound according to claim 1 or a salt thereof, wherein X represents NH; Y represents CO; Z¹, Z², Z³, and Z⁴ each independently represent CH; W² represents N; W¹, W³, and W⁴ each independently represent CH; and an amino group is located at position 4 (such compound being referred to as "Compound 14" herein).
(16) An analgesic agent comprising, as an active ingredient, the compound according to any of (1) to (15) or a pharmaceutically acceptable salt thereof.
(17) An antiinflammatory agent comprising, as an active ingredient, the compound according to any of (1) to (15) or a pharmaceutically acceptable salt thereof.
(18) An antitumor agent comprising, as an active ingredient, the compound according to any of (1) to (15) or a pharmaceutically acceptable salt thereof.
(19) An antiplatelet aggregation agent comprising, as an active ingredient, the compound according to any of (1) to (15) or a pharmaceutically acceptable salt thereof.
(20) A cyclooxygenase-1-selective inhibitor comprising, as an active ingredient, the compound according to any of (1) to (15) or a pharmaceutically acceptable salt thereof.
(21) A method for relieving pain in a subject in need of analgesia comprising administering an effective amount of the compound according to any of (1) to (15) or a pharmaceutically acceptable salt thereof to the subject.
(22) A method for preventing or treating an inflammatory disease in a subject in need of prevention or treatment of an inflammatory disease comprising administering an effective amount of the compound according to any of (1) to (15) or a pharmaceutically acceptable salt thereof to the subject.
(23) A method for preventing or treating cancer in a subject in need of prevention or treatment of cancer comprising administering an effective amount of the compound according to any of (1) to (15) or a pharmaceutically acceptable salt thereof to the subject.
(24) A method for suppressing platelet aggregation *in vitro* or *in vivo* comprising adding an effective amount of the compound according to any of (1) to (15) or a pharmaceutically acceptable salt thereof to an *in vitro* or *in vivo* environment in which platelets are present.
(25) A method for selectively inhibiting cyclooxygenase-1 *in vitro* or *in vivo* comprising adding an effective amount of the compound according to any of (1) to (15) or a pharmaceutically acceptable salt thereof to an *in vitro* or *in vivo* environment in which cyclooxygenase-1 is present.
(26) A method for treating, preventing, or ameliorating a disease or symptom that can be ameliorated via selective inhibition of cyclooxygenase-1 in a subject in need of treatment, prevention, or amelioration of such disease or symptom comprising administering an effective amount of the compound according to any of (1) to (15) or a pharmaceutically acceptable salt thereof to the subject.
(27) An analgesic pharmaceutical composition comprising an effective amount of the compound according to any of (1) to (15) or a pharmaceutically acceptable salt thereof.
(28) A pharmaceutical composition for preventing or treating an inflammatory disease comprising an effective amount of the compound according to any of (1) to (15) or a pharmaceutically acceptable salt thereof.
(29) A pharmaceutical composition for preventing or treating cancer comprising an effective amount of the compound according to any of (1) to (15) or a pharmaceutically acceptable salt thereof.
(30) A composition or pharmaceutical composition for suppressing platelet aggregation *in vitro* or *in vivo* comprising an effective amount of the compound according to any of (1) to (15) or a pharmaceutically acceptable salt thereof.
(31) A composition or pharmaceutical composition for selectively inhibiting cyclooxygenase-1 *in vitro* or *in vivo* comprising an effective amount of the compound according to any of (1) to (15) or a pharmaceutically acceptable salt thereof.
(32) A pharmaceutical composition for treating, preventing, or ameliorating a disease or symptom that can be ameliorated via selective inhibition of cyclooxygenase-1 comprising an effective amount of the compound according to any of (1) to (15) or a pharmaceutically acceptable salt thereof.

### Effects of the Invention

The compound of the present invention is a cyclooxygenase-1 (COX-1)-selective inhibitor comprising a novel and chemically stable skeleton. Because of its simple structure, mass synthesis thereof can be realized with a high yield through a small number of steps. Significant analgesic effects have been demonstrated through mouse-based experiments. Also, the compound of the present invention was confirmed not to cause gastrointestinal trouble or to be toxic. Unlike Mofezolac, which is a known cyclooxygenase-1-selective inhibitor, the compound of the present invention is a basic compound. Also, the compound of the present invention is useful as a reagent for research.

This description includes part or all of the contents as disclosed in the description and/or drawings of Japanese Patent Application No. 2006-242861, which is a priority document of the present application.

### Preferred Embodiments of the Invention

The compound of the present invention may be any of the compounds represented by the above formula.

In the formula, W¹, W², W³, and W⁴ are preferably combined with one another such that the aromatic ring to which they belong can be a benzene ring, pyridine ring, or pyrimidine ring, and Z¹, Z², Z³, and Z⁴ are preferably combined with one another such that the aromatic ring to which they belong can be a benzene ring, pyridine ring, or pyrimidine ring.

A particularly preferable compound of the present invention is represented by the above formula, wherein either X or Y represents CO and the other represents NH; W¹ and W² each independently represent CH or N (provided that W¹ and W² would not simultaneously represent N) and W³ and W⁴ each independently represent CH; Z¹ represents CH or N and Z², Z³, and Z⁴ each independently represent CH; and an amino group is located at position 4, 5, or 6 or a salt thereof.

A particularly preferable compound of the present invention or a salt thereof is represented by the above formula, wherein either X or Y represents CO and the other represents NH; W² represents CH or N, W¹, W³ and W⁴ each independently represent CH; Z¹ represents CH or N and Z², Z³, and Z⁴ each independently represent CH; and an amino group is located at position 4, 5, or 6. A compound or a salt thereof comprising an amino group at position 4 is the most preferable.

A particularly preferable compound of the present invention or a salt thereof is represented by the above formula, wherein X represents CO; Y represents NH; W¹, W², W³, and W⁴ each independently represent CH; Z¹ represents CH or N and Z², Z³, and Z⁴ each independently represent CH; and an amino group is located at position 4, 5, or 6.

A particularly preferable compound of the present invention or a salt thereof is represented by the above formula, wherein X represents NH, Y represents CO; W¹ and W² each independently represent CH or N (provided that W¹ and W² would not simultaneously represent N) and W³ and W⁴ each independently represent CH; Z¹ represents CH or N and Z², Z³, and Z⁴ each independently represent CH; and an amino group is located at position 4, 5, or 6.

Specific examples of preferable compounds of the present invention include compounds 1 to 14 above.

The fact that all compounds represented by the formula of the present invention have activity of COX-1-selective inhibition can be rationally explained based on the results of experiments disclosed herein and general technical knowledge. The grounds therefor are as described below. (i) Aromatic rings included in the compound of the present invention (e.g., a benzene ring, a pyridine ring, and a pyrimidine ring) are known to be conformationally similar and compatible to each other. In the examples provided herein, activity of COX-1-selective inhibition is observed regardless of aromatic ring type. (ii) Similar activities of COX-1-selective inhibition are confirmed in the examples regardless of whether -X-Y-represents -CO-NH- or -NH-CO-. (iii) Activity of COX-1-selective inhibition is observed in a compound comprising an amino group at the o-, m-, or p-position.

Since COX-1 inhibitory activity was observed in compounds via *in vitro* assay, a compound represented by the above formula is considered to be useful as a molecular biological reagent against COX-1.

The compound of the present invention can be used in the form of a pharmaceutically acceptable salt. Examples of pharmaceutically acceptable salts include inorganic acid salts, such as hydrochloride, sulfate, bromate, iodate, phosphate, nitrate, and sulfate, and organic acid salts, such as acetate and citrate.

The compound of the present invention or a salt thereof may be in the form of, for example, a hydrate with water or a solvate with a lower alcohol. The compound of the present invention or a salt thereof includes a hydrate or solvate thereof.

Subsequently, methods for synthesizing the compound of the present invention in a case in which -X-Y- represents -CO-NH- and in a case in which -X-Y- represents -NH-CO- are separately described.

A compound in which -X-Y- represents -CO-NH- can be synthesized in accordance with the scheme shown below. In the scheme, definitions of W¹, W², W³, W⁴, Z¹, Z², Z³, and Z⁴ and the sites thereof to which amino groups bind are as described above.

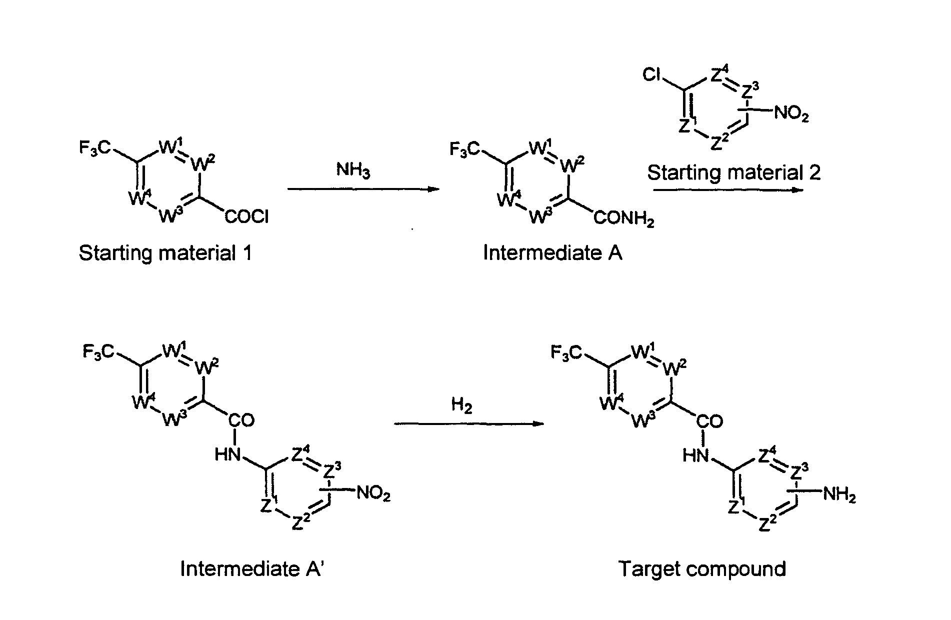

An example of a synthesis procedure in accordance with the above scheme is described below. Starting materials 1 and 2 may be commercially available reagents.

Aqueous ammonia is added to starting material 1 at room temperature with stirring, and the reaction is then allowed to proceed for approximately 3 hours. Subsequently, the reaction mixture is transferred into water, followed by extraction with ethyl acetate. The ethyl acetate layer is washed with water and with saturated saline and is then dried over magnesium sulfate. The resultant is then filtered, and a crude product is obtained via removal of a solvent by distillation under reduced pressure. The resulting crude product is recrystallized with a mixed solvent of ethyl acetate/hexane to obtain intermediate A.

Starting material 2, cesium carbonate, 4,5-bis(diphenylphosphino)-9,9-dimethylxanthine, tris(dibenzylideneacetone)dipalladium, and dioxane are added to intermediate A, and air is substituted with argon, followed by heating under reflux overnight. The reaction mixture is subjected to filtration through Celite, transferred into water, and then extracted with ethyl acetate. The ethyl acetate layer is washed with water and with saturated saline and is then dried over magnesium sulfate. The resultant is then filtered, and a crude product is obtained via removal of a solvent by distillation under reduced pressure. Subsequently, a target fraction is separated via flash column chromatography (ethyl acetate:hexane = 1:2) and treated via removal of a solvent by distillation under reduced pressure to obtain a crude product. The resulting crude product is recrystallized with a mixed solvent of ethyl acetate/hexane to obtain intermediate A'.

Intermediate A' is dissolved in ethyl acetate, a catalytic amount of palladium carbon is added thereto, the mixture is stirred under a hydrogen balloon at room temperature, and the reaction is then allowed to proceed overnight. The reaction mixture is subjected to filtration through Celite, and a crude product is then obtained via removal of a solvent by distillation under reduced pressure. The resulting crude product is recrystallized with a mixed solvent of ethyl acetate/hexane to obtain a target compound.

A compound in which -X-Y- represents -NH-CO- can be synthesized in accordance with the scheme shown below. In the scheme, definitions of W¹, W², W³, W⁴, Z¹, Z², Z³, and Z⁴ and the sites thereof to which amino groups bind are as described above.

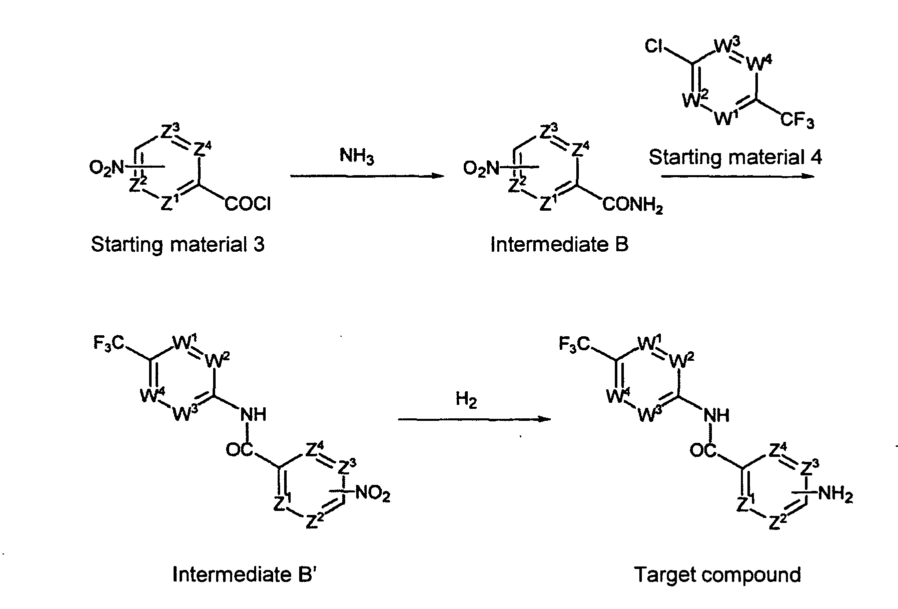

An example of a synthesis procedure in accordance with the above scheme is described below. Starting materials 3 and 4 may be commercially available reagents.

Aqueous ammonia is added to starting material 3 at room temperature with stirring, and the reaction is then allowed to proceed for approximately 3 hours. Subsequently, the reaction mixture is transferred into water and then extracted with ethyl acetate. The ethyl acetate layer is washed with water and with saturated saline and is then dried over magnesium sulfate. The resultant is then filtered, and a crude product is obtained via removal of a solvent by distillation under reduced pressure. The resulting crude product is recrystallized with a mixed solvent of ethyl acetate/hexane to obtain intermediate B.

Starting material 4, cesium carbonate, 4,5-bis(diphenylphosphino)-9,9-dimethylxanthine, tris(dibenzylideneacetone)dipalladium, and dioxane are added to intermediate B, and air is substituted with argon, followed by heating under reflux overnight. The reaction mixture is subjected to filtration through Celite, transferred into water, and then extracted with ethyl acetate. The ethyl acetate layer is washed with water and with saturated saline and is then dried over magnesium sulfate. The resultant is then filtered, and a crude product is obtained via removal of a solvent by distillation under reduced pressure. Subsequently, a target fraction is separated via flash column chromatography (ethyl acetate:hexane = 1:2) and treated via removal of a solvent by distillation under reduced pressure to obtain a crude product. The resulting crude product is recrystallized with a mixed solvent of ethyl acetate/hexane to obtain intermediate B'.

Intermediate B' is dissolved in ethyl acetate, a catalytic amount of palladium carbon is added thereto, the mixture is stirred under a hydrogen balloon at room temperature, and the reaction is then allowed to proceed overnight. The reaction mixture is subjected to filtration through Celite, and a crude product is then obtained via removal of a solvent by distillation under reduced pressure. The resulting crude product is recrystallized with a mixed solvent of ethyl acetate/hexane to obtain a target compound.

The compound of the present invention can be administered to a subject, particularly to a mammalian, such as a human, mouse, rat, or bovine, and more particularly to a human, to selectively inhibit COX-1 in such mammalian. Thus, administration of an effective amount of the compound of the present invention to a subject enables treatment, prevention, or amelioration of a disease or symptom that can be treated, prevented, or ameliorated via selective inhibition of COX-1. Examples of such disease or symptom include, but are not limited to, cancer (particularly colon cancer), pain, inflammation, heart disease resulting from clots, and cerebral thrombosis.

The compound of the present invention can be administered to a mammalian in the form of a pharmaceutical composition comprising a pharmaceutically acceptable carrier or an additive together with the compound. Examples of such carrier and additive include water, pharmaceutically acceptable organic solvent, collagen, polyvinyl alcohol, polyvinylpyrrolidone, carboxyvinyl polymer, sodium alginate, water-soluble dextran, sodium carboxymethyl starch, pectin, xanthan gum, gum Arabic, casein, gelatin, agar, glycerin, propylene glycol, polyethylene glycol, vaseline, paraffin, stearyl alcohol, stearic acid, human serum albumin, mannitol, sorbitol, lactose, a pharmaceutically acceptable surfactant, and an artificial cell structure, such as a liposome. An additive to be used is adequately selected from among the above substances in accordance with the dosage form of the pharmaceutical composition, or combinations of more than one substance are selected.

The compound of the present invention can be administered orally or parenterally.

When the compound of the present invention is orally administered, the compound can be prepared in the form of, for example, a solid preparation, such as a tablet, granules, powder, or pill, or a liquid preparation, such as a liquid or syrup. Particularly, granules and powder can be prepared into a unit dosage form as a capsule. A liquid preparation may be in the form of a dry product, which is to be resolubilized at the time of use.

A solid preparation can comprise an additive that is commonly used for a pharmaceutical preparation, such as a binder, an excipient, a lubricant, a disintegrator, or a moistening agent. A liquid preparation can comprise an additive that is commonly used for a pharmaceutical preparation, such as a stabilizer, a buffer, a flavor, a preservative, an aroma chemical, or a coloring agent.

When the compound is parenterally administered, the compound may be in the form of, for example, an injection, suppository, or skin external preparation. For example, an injection is prepared by dissolving or suspending the compound of the present invention in a solution, suspension, emulsion, or the like, and it is generally provided in the form of a unit dose ampoule or multiple-dose container. Also, an injection preparation may be a powder that is redissolved in an adequate carrier, such as sterilized water containing no pyogenic substance, at the time of use. Examples of routes of injection include intravenous drip injection, intravenous injection, intramuscular injection, intraperitoneal injection, hypodermic injection, and percutaneous injection. Such parenteral dosage forms generally comprise an additive, such as an emulsion or suspension that is generally used for pharmaceuticals.

The amount of the compound of the present invention in a pharmaceutical composition and the dosage of the compound of the present invention applied to a patient are not particularly limited, provided that the target disease or symptom can be treated, prevented, or ameliorated. Such amounts can be adequately determined in accordance with an intended application, a dosage form, a route of administration, or other conditions. For example, a dosage may be 1 to 50 mg per kg of the body weight of a patient per day.

The compound of the present invention can also be used as an active ingredient of a COX-1-selective inhibitor, which is not limited to a given target of treatment. The COX-1-selective inhibitor of the present invention can be prepared in the same manner as the above pharmaceutical composition. The COX-1-selective inhibitor of the present invention can be used as a reagent for biological research.

Hereafter, the present invention is described in greater detail with reference to the examples, although the present invention is not limited thereto.

### Examples

### [Example 1]

### Synthesis of compound 1

In accordance with the following scheme of synthesis, 4-(trifluoromethyl)-benzoyl chloride was converted into compound C and then into compound C' to prepare compound 1.

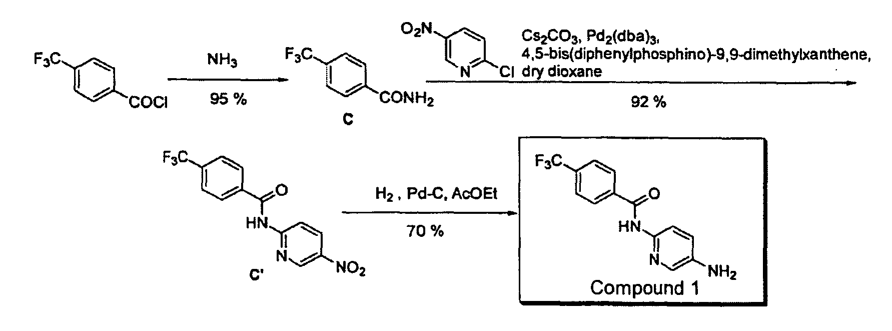

### Synthesis of compound C

Aqueous ammonia (2 ml) was added to 4-(trifluoromethyl)-benzoyl chloride (417 mg, 2 mmol) at room temperature with stirring, and the reaction was allowed to proceed for 3 hours. The completion of the reaction was confirmed using a TLC plate (ethyl acetate:hexane = 1:2), and the reaction product was introduced into 10 ml of water, followed by extraction with ethyl acetate (10 ml × 3). The ethyl acetate layer was washed with water (10 ml × 2) and with saturated saline (10 ml), dried over magnesium sulfate, and then filtered to obtain a crude product via removal of a solvent by distillation under reduced pressure (355 mg, 2.0 mmol). The resultant was recrystallized with a mixed solvent of ethyl acetate/hexane to obtain compound C (white, needle-like crystal, 359 mg).
Yield: 95%; m.p.: 186.5-187.5°C
¹H-NMR (300 MHz, DMSO-d6) δ: 7.63, 8.19 (both br s, both 1H, NH or OH), 8.06 (d, 2H, J = 8.0 Hz, Ar-H), 7.84 (d, 2H, J = 8.0 Hz, Ar-H)

### Synthesis of compound C'

2-Chloro-5-nitropyridine (295 mg, 1.9 mmol), cesium carbonate (668 ml, 2.1 mmol), 4,5-bis(diphenylphosphino)-9,9-dimethylxanthine (214 mg, 0.4 mmol), tris(dibenzylideneacetone)dipalladium (82 mg, 0.1 mmol), and dioxane (15 ml) were added to compound C (352 mg, 1.9 mmol), and air was substituted with argon, followed by heating under reflux overnight. The completion of the reaction was confirmed using a TLC plate (ethyl acetate:hexane = 1:2), the reaction product was filtered through Celite, and the resultant was then introduced into 70 ml of water, followed by extraction with ethyl acetate (70 ml × 3). The ethyl acetate layer was washed with water (70 ml × 2) and with saturated saline (70 ml), dried over magnesium sulfate, and then filtered to obtain a crude product via removal of a solvent by distillation under reduced pressure (852 mg, 2.7 mmol). Subsequently, a target fraction was separated via flash column chromatography (ethyl acetate:hexane = 1:2) to obtain a crude product via removal of a solvent by distillation under reduced pressure (495 mg). The resultant was recrystallized with a mixed solvent of ethyl acetate/hexane to obtain compound C' (white, needle-like crystal, 530 mg).
Yield: 92%; m.p.: 115-116°C
¹H-NMR (300 MHz, DMSO-d6) δ: 11.82 (s, 1H, NH), 8.69 (dd, 1H, J = 9.0, 3.0 Hz, Ar-H), 8.45 (dd, 1H, J = 9.0, 1.0 Hz, Ar-H), 8.22 (d, 2H, J = 8.0 Hz, Ar-H), 7.91 (m, 3H, Ar-H)

### Synthesis of target compound 1

Compound C' (500 mg, 1.6 mmol) was dissolved in ethyl acetate (4 ml), a spoonful of palladium carbon was added thereto, the mixture was stirred under a hydrogen balloon at room temperature, and the reaction was allowed to proceed overnight. The completion of the reaction was confirmed using a TLC plate (ethyl acetate:hexane = 1:2) and filtered through Celite to obtain a crude product via removal of a solvent by distillation under reduced pressure (413 mg, 1.5 mmol). The resultant was recrystallized with a mixed solvent of ethyl acetate/hexane to obtain target compound 1 (white, plate-like crystal, 314 mg).
Yield: 70%; m.p.: 140.5-141.5°C
¹H-NMR (300 MHz, DMSO-d6) δ: 10.62 (s, 1H, NH), 8.16 (d, 2H, J = 8.0 Hz, Ar-H), 7.84 (d, 2H, J = 9.0 Hz, Ar-H), 7.75 (dd, 2H, J = 3.0, 1.0 Hz, Ar-H), 7.03 (dd, 1H, J = 9.0, 3.0 Hz, Ar-H), 5.22 (s, 2H, NH₂)

### Synthesis of compound 2

Compound 2 was prepared in the same manner as in the case of compound 1, except that 2-chloro-3-nitropyridine was used instead of 2-chloro-5-nitropyridine.
Yield: 78%; m.p.: 207-208°C
¹H-NMR (300 MHz, DMSO-d6) δ: 10.57 (s, 1H, NH), 8.20 (d, 2H, J = 8.0 Hz, Ar-H), 7.89 (d, 2H, J = 8.0 Hz, Ar-H), 7.72 (m, 1H, Ar-H), 7.16 (dd, 1H, J = 8.0, 1.5 Hz, Ar-H), 7.08 (m, 1H, Ar-H), 5.10 (s, 2H, NH₂)

### Synthesis of compound 3

In accordance with the following scheme of synthesis, 4-nitroaniline was converted into compound D to prepare compound 3.

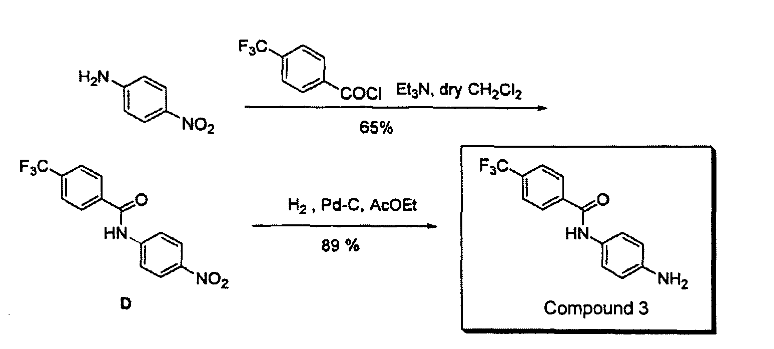

### Synthesis of compound D

Dry dichloromethane (20 ml), triethylamine (0.3 ml 2 mmol), and 4-(trifluoromethyl)-benzoyl chloride (0.3 ml, 2 mmol) were successively added to 4-nitroaniline (276 mg, 2 mmol) at room temperature with stirring, and the reaction was allowed to proceed for 1.5 hours. The completion of the reaction was confirmed using a TLC plate (ethyl acetate:hexane = 1:4), and the reaction product was introduced into 50 ml of water, followed by extraction with dichloromethane (50 ml × 3). The dichloromethane layer was washed with water (50 ml × 2), dried over magnesium sulfate, and then filtered to obtain a crude product via removal of a solvent by distillation under reduced pressure (700 mg, 2 mmol). Subsequently, the target fraction was separated via flash column chromatography (ethyl acetate:hexane = 1:6) to obtain a crude product via removal of a solvent by distillation under reduced pressure (410 mg). The resultant was recrystallized with a mixed solvent of ethyl acetate/hexane to obtain target compound D (white, needle-like crystal, 404 mg).
Yield: 65%; m.p.: 196-198°C
¹H-NMR (300 MHz, DMSO-d6) δ: 11.10 (s, 1H, NH), 8.29 (d, 2H, J = 9.5 Hz, Ar-H), 8.18 (d, 2H, J = 8.0 Hz, Ar-H), 8.07 (d, 2H, J = 9.5 Hz, Ar-H), 7.95 (d, 2H, J = 8.0 Hz, Ar-H)

### Synthesis of target compound 3

Compound D (345 mg, 1.1 mmol) was dissolved in ethyl acetate (20 ml), a spoonful of palladium carbon was added thereto, the mixture was stirred under hydrogen baloon at room temperature, and the reaction was allowed to proceed for 2 hours. The completion of the reaction was confirmed using a TLC plate (ethyl acetate:hexane = 1:2) and filtered through Celite to obtain a crude product via removal of a solvent by distillation under reduced pressure (303 mg, 1.0 mmol). The resultant was recrystallized with a mixed solvent of ethyl acetate/hexane to obtain target compound 3 (white, powdery crystal, 279 mg).
Yield: 89%; m.p.: 223-224°C
¹H-NMR (300 MHz, DMSO-d6) δ: 10.09 (s, 1H, NH), 8.11 (d, 2H, J = 8.0 Hz, Ar-H), 7.87 (d, 2H, J = 8.0 Hz, Ar-H), 7.38 (s, 2H, J = 9.0 Hz, Ar-H), 6.55 (d, 2H, J = 9.0 Hz, Ar-H), 4.96 (s, 2H, NH₂)

### Synthesis of compound 4

Compound 4 was prepared in the same manner as in the case of compound 3, except that 3-nitroaniline was used instead of 4-nitroaniline.
Yield: 74%; m.p.: 159-162°C
¹H-NMR (300 MHz, DMSO-d6) δ: 10.16 (s, 1H, NH), 8.10 (d, 2H, J = 8.5 Hz, Ar-H), 7.89 (d, 2H, J = 8.5 Hz, Ar-H), 7.09 (dd, 1H, J = 2.2, 1.8 Hz, Ar-H), 6.97 (t, 1H, J = 8.0 Hz, Ar-H), 6.86 (d, 1H, J = 8.0 Hz, Ar-H), 6.3 (ddd, 1 H, J = 8.0, 2.2, 1.8 Hz, Ar-H), 5.12 (s, 2H, NH₂)

### Synthesis of compound 5

Compound 5 was prepared in the same manner as in the case of compound 3, except that 4-aminobenzotrifluoride and 4-nitrobenzoyl chloride were used instead of 4-nitroaniline and 4-(trifluoromethyl)-benzoyl chloride, respectively.
Yield: 75%; m.p.: 232-233°C
¹H-NMR (300 MHz, DMSO-d6) δ: 10.10 (s, 1H, NH), 7.98 (d, 2H, J = 8.5 Hz, Ar-H), 7.73 (dd, 2H, J = 7.0, 2.0 Hz, Ar-H), 7.66 (d, 2H, J = 8.5 Hz, Ar-H), 6.63 (dd, 2H, J = 7.0, 2.0 Hz, Ar-H), 5.80 (s, 2H, NH₂)

### Synthesis of compound 6

Compound 6 was prepared in the same manner as in the case of compound 3, except that 4-aminobenzotrifluoride and 3-nitrobenzoyl chloride were used instead of 4-nitroaniline and 4-(trifluoromethyl)-benzoyl chloride, respectively.
Yield: 89%; m.p.: 213-216°C
¹H-NMR (300 MHz, DMSO-d6) δ: 10.20 (s, 1H, NH), 8.00 (d, 2H, J = 8.5 Hz, Ar-H), 7.70 (d, 2H, J = 8.5 Hz, Ar-H), 7.17 (t, 1H, J = 7.5 Hz, Ar-H), 7.05 (m, 2H, Ar-H), 6.77 (m, 1H, Ar-H), 5.40 (s, 2H, NH₂)

### Synthesis of compound 10

In accordance with the following scheme of synthesis, 2-bromo-5-nitropyridine was converted into compound E and then into compound E' to prepare compound 10.

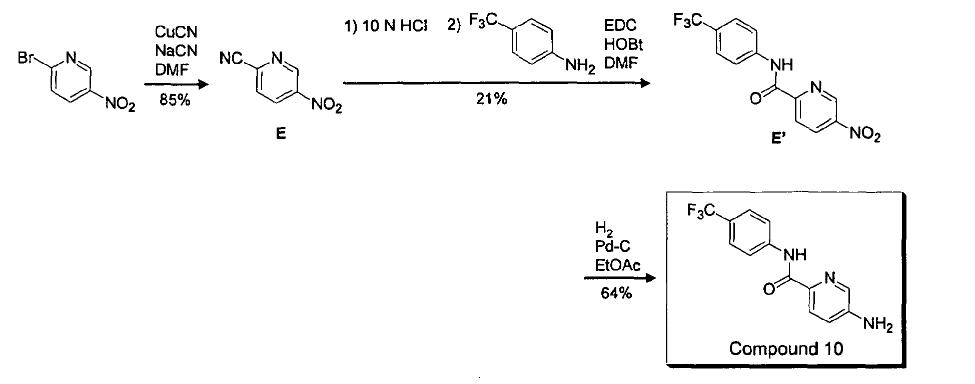

### Synthesis of compound E

Copper cyanide (I) (662 mg, 7.34 mmol) and sodium cyanide (I) (242 mg, 4.93 mmol) were dissolved in N,N-dimethylformamide (7.5 ml), and the solution was stirred at 150°C for 25 minutes. A solution of 2-bromo-5-nitropyridine (113 mg, 0.700 mmol) completely dissolved in N,N-dimethylformamide (2.5 ml) at 100°C was added to the solution, and the mixture was stirred with heating at 150°C for 100 minutes. The completion of the reaction was confirmed using a TLC plate (ethyl acetate:hexane = 1:3), and the reaction was terminated with 10 ml of an aqueous solution of 1 M monopotassium phosphate, followed by extraction with ethyl acetate (50 ml × 3). The ethyl acetate layer was washed with water (100 ml) and with saturated saline (100 ml). The resultant was dried over magnesium sulfate and filtered, followed by removal of a solvent by distillation under reduced pressure. The product was separated via flash column chromatography (ethyl acetate:hexane = 1:7 → 1:3) to obtain a yellow, oil-like target compound E (624 mg, 4.19 mmol).
Yield: 85%
¹H-NMR (300 MHz, CDCl₃) δ: 9.54 (dd, 1H, J = 2.5, 0.7 Hz, Py-H), 8.68 (dd, 1H, J = 8.4, 2.5 Hz, Py-H), 7.98 (dd, 1H, J = 8.4, 0.7 Hz, Py-H)

### Synthesis of compound E'

10 N HCl (3 ml) was added to compound E (247 mg, 1.66 mmol), and air was substituted for argon, followed by heating under reflux for 19 hours. The completion of the reaction was confirmed using a TLC plate (ethyl acetate:hexane = 1:3), and a crude product (351 mg) was obtained via removal of a solvent by distillation under reduced pressure. 4-Aminobenzotrifluoride (113 mg, 0.700 mmol), 1-ethyl-3-(3-dimethylaminopropyl)carbodiimide hydrochloride (134 mg, 0.700 mmol), and 1-hydroxybenzotriazole monohydrate (95 mg, 0.700 mmol) were added to the crude product (100 mg, 0.595 mmol), dissolved in N,N-dimethylformamide (3 ml), and then stirred at room temperature for a day. The completion of the reaction was confirmed using a TLC plate (ethyl acetate:hexane = 1:1), and the reaction was terminated with water (20 ml), followed by extraction with ethyl acetate (20 ml × 2). The ethyl acetate layer was washed with water (20 ml) and with saturated saline (20 ml), dried over magnesium sulfate, and then filtered, followed by removal of a solvent by distillation under reduced pressure. The resultant was separated via flash column chromatography (ethyl acetate:hexane = 1:5) to obtain white, powdery target compound E' (39 mg, 0.125 mmol).
Yield: 21 %
¹H-NMR (300 MHz, CDCl₃) δ: 10.04 (s, 1H, NH), 9.46 (dd, 1H, J = 2.5, 0.7 Hz, Py-H), 8.74 (dd, 1H, J = 8.6, 2.5 Hz, Py-H), 8.54 (dd, 1H, J = 8.6, 0.7 Hz, Py-H), 7.92 (d, 2H, J = 8.4 Hz, Ar-H), 7.68 (d, 2H, J = 8.4 Hz, Ar-H)

### Synthesis of target compound 10

Compound E' (6 mg, 0.019 mmol) was dissolved in ethyl acetate (0.5 ml), a spoonful of palladium carbon was added thereto, and the resultant was stirred under a hydrogen atmosphere at room temperature for 2 hours. The completion of the reaction was confirmed using a TLC plate (ethyl acetate:hexane = 1:2) and filtered through Celite to obtain a crude product via removal of a solvent by distillation under reduced pressure (6 mg). The resultant was separated via flash column chromatography (ethyl acetate:hexane = 1:1 → 3:1) to obtain white, powdery target compound 10 (3 mg, 0.012 mmol).
Yield: 64%; m.p.: 155-156°C
¹H-NMR (300 MHz, CDCl₃) δ: 9.94 (s, 1H, NH), 8.09 (d, 1H, J = 8.5 Hz, Py-H), 8.01 (dd, 1H, J = 2.8, 0.7 Hz, Py-H), 7.88 (d, 2H, J = 8.5 Hz, Ar-H), 7.62 (d, 2H, J = 8.5 Hz, Ar-H), 7.09 (dd, 1H, J = 8.5, 2.8 Hz, Py-H), 4.13 (s, 2H, NH₂)

### Synthesis of compound E"

Compound E"was prepared in the same manner as in the case of compound E' except that 5-amino-2-trifluoromethylpyridine was used instead of 4-aminobenzotrifluoride.
Yield: 40%
¹H-NMR (300 MHz, CDCl₃) δ: 10.25 (s, 1H, NH), 9.48 (dd, 1H, J = 2.4, 0.7 Hz, Py-H), 8.93 (d, 1H, J = 2.4 Hz, Py-H), 8.76 (dd, 1H, J = 8.5, 2.4 Hz, Py-H), 8.63 (dd, 1H, J = 8.2, 2.4 Hz, Py-H), 8.55 (dd, 1H, J = 8.5, 0.7 Hz, Py-H), 7.77 (d, 1H, J = 8.2, Py-H)

### Synthesis of compound 11

Compound 11 was prepared in the same manner as in the case of compound 10 except that compound E" was used instead of compound E'.
Yield: 67%; m.p.: 212.5-213°C
¹H-NMR (300 MHz, DMSO-d6) δ: 10.84 (s, 1H, NH), 9.21 (d, 1H, J = 2.0 Hz, Py-H), 8.60 (dd, 1H, J = 8.4, 2.0 Hz, Py-H), 8.04 (d, 1 H, J = 2.6 Hz, Py-H), 7.87 (dd, 2H, J = 8.4, 1.5 Hz, Py-H), 7.05 (dd, 1H, J = 8.4, 2.6 Hz, Py-H), 6.23 (s, 2H, NH₂)

### Synthesis of compound E"'

Compound E"' was prepared in the same manner as in the case of compound E' except that 2-amino-5-trifluoromethylpyridine was used instead of 4-aminobenzotrifluoride.
Yield: 4%
¹H-NMR (500 MHz, CDCl₃) δ: 10.55 (br s, 1H, NH), 9.48 (dd, 1H, J = 2.4, 0.6 Hz, Py-H), 8.73 (dd, 1H, J = 8.6, 2.4 Hz, Py-H), 8.66 (dd, 1H, J = 1.5, 0.9 Hz, Py-H), 8.54 (m, 2H, Py-H), 8.03 (dd, 1H, J = 8.6, 2.4 Hz, Py-H)

### Synthesis of compound 12

Compound 12 was prepared in the same manner as in the case of compound 10 except that compound E"' was used instead of compound E'.
Yield: 52%; m.p.: 214.5-216.5°C
¹H-NMR (500 MHz, CDCl₃) δ: 10.51 (s, 1H, NH), 8.59 (m, 1H, Py-H), 8.54 (d, 1H, J = 8.9 Hz, Py-H), 8.08 (d, 1H, J = 8.6 Hz, Py-H), 8.04 (d, 2H, J = 2.7 Hz, Py-H), 7.95 (d, 1H, J = 8.9 Hz, Py-H), 7.08 (dd, 1H, J = 8.2, 2.7 Hz, Py-H), 4.16 (s, 2H, NH₂)

### Synthesis of compound 13

In accordance with the following scheme of synthesis, 4-(trifluoromethyl)-benzoyl chloride was converted into compound F and then into compound F' to prepare compound 13.

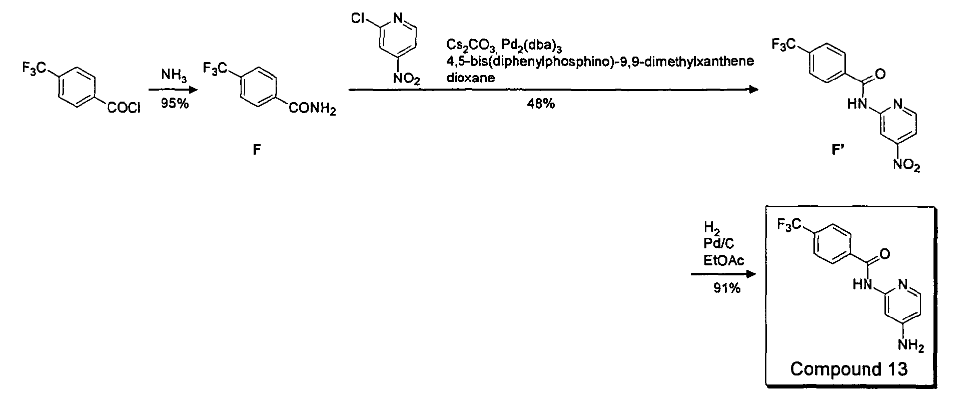

### Synthesis of compound F

Aqueous ammonia (2 ml) was added to 4-(trifluoromethyl)-benzoyl chloride (417 mg, 2 mmol), and the reaction was allowed to proceed at room temperature for 3 hours. The completion of the reaction was confirmed using a TLC plate (ethyl acetate:hexane = 1:2), and the reaction was terminated with water (10 ml), followed by extraction with ethyl acetate (10 ml × 3). The ethyl acetate layer was washed with water (10 ml × 2) and with saturated saline (10 ml), dried over magnesium sulfate, and then filtered to obtain a crude product via removal of a solvent by distillation under reduced pressure (355 mg, 2.0 mmol). The resultant was recrystallized with a mixed solvent of ethyl acetate/hexane to obtain compound F (white, needle-like crystal, 359 mg).
Yield: 95%; m.p.: 186.5-187.5°C
¹H-NMR (300 MHz, DMSO-d6) δ: 7.63, 8.19 (both br s, both 1H, NH or OH), 8.06 (d, 2H, J = 8.0 Hz, Ar-H), 7.84 (d, 2H, J = 8.0 Hz, Ar-H)

### Synthesis of compound F'

2-Chloro-4-nitropyridine (328 mg, 2.07 mmol), cesium carbonate (237 mg, 0.41 mmol), 4,5-bis(diphenylphosphino)-9,9-dimethylxanthine (92 mg, 0.10 mmol), tris(dibenzylideneacetone)dipalladium (743 mg, 2.28 mmol), and dioxane(15 ml) were added to compound F (392 mg, 2.07 mmol), and the resultant was subjected to heating under reflux under an argon atmosphere overnight. The termination of the reaction was confirmed using a TLC plate (ethyl acetate:hexane = 1:2), the reaction product was filtered through Celite, and the resultant was added to water (150 ml) to terminate the reaction, followed by extraction with ethyl acetate (70 ml × 3). The ethyl acetate layer was washed with water (70 ml × 2) and with saturated saline (70 ml), dried over magnesium sulfate, and then filtered to obtain a crude product via removal of a solvent by distillation under reduced pressure (1.024 g). The resultant was separated via flash column chromatography (ethyl acetate:hexane = 1:3) to obtain white, powdery target compound F' (310 mg, 1.00 mmol).
Yield: 48%; m.p.: 207.0-208.0°C
¹H-NMR (DMSO-d6) δ: 11.71 (s, 1H, NH), 8.94 (dd, 1H, J = 2.0, 1.0 Hz, Ar-H), 8.77 (dd, 1H, J = 5.5, 1.0 Hz, Ar-H), 8.23 (d, 2H, J = 8.0 Hz, Ar-H), 7.92 (dd, 3H, J = 5.5, 2.0 Hz, Ar-H)

### Synthesis of target compound 13

Compound F' (310 mg, 1.00 mmol) was dissolved in ethyl acetate (10 ml), a spoonful of palladium carbon was added thereto, and the reaction was allowed to proceed under a hydrogen atmosphere at room temperature for 3 hours. The completion of the reaction was confirmed using a TLC plate (ethyl acetate:hexane = 1:2) and the resultant was filtered through Celite to obtain a crude product via removal of a solvent by distillation under reduced pressure (255 mg). The resultant was recrystallized with a mixed solvent of ethyl acetate/hexane to obtain target compound 13 (white, powdery crystal, 191 mg).
Yield: 91%; m.p.: 221.0-223.0°C
¹H-NMR (DMSO-d6) δ: 10.53 (s, 1H, NH), 8.15 (d, 2H, J = 8.0 Hz, Ar-H), 7.85 (d, 2H, J = 8.0 Hz, Ar-H), 7.80 (d, 1H, J = 6.0 Hz, Py-H), 7.43 (d, 1H, J = 2.0 Hz, Py-H), 6.30 (dd, 1H, J = 6.0, 2.0 Hz, Py-H), 6.17 (s, 2H, NH₂)

### Synthesis of target compound 14

Compound 14 was prepared in the same manner as in the case of compound 13 except that 4-nitrobenzoyl chloride and 2-chloro-5-(trifluoromethyl)-pyridine were used instead of 4-(trifluoromethyl)-benzoyl chloride and 2-chloro-4-nitropyridine, respectively.
Yield: 94%; m.p.: 167.5-169.0°C
¹H NMR (300MHz, DMSO-d6) δ: 10.68 (s, 1H, NH), 8.71 (s, 1H, Py-H), 8.38 (d, 1H, J = 8.8Hz, Py-H), 8.17 (d, 1H, J = 8.8 Hz, Py-H), 7.81 (d, 2H, J = 8.7 Hz, Ar-H), 6.58 (d, 2H, J = 8.7 Hz, Ar-H), 5.91 (s, 1H, NH₂)

### [Example 2]

### Evaluation of activity

### In vitro test (1)

The Cayman Cyclooxygenase Inhibitor Screening Assay Kit (760111) was used to determine the inhibitory activities (%) of 100 µM compounds 1 to 6, 10, 11, and 14 against cyclooxygenase-1 (COX-1) and cyclooxygenase 2 (COX-2).

Compound 1 exhibited particularly high inhibitory activity against COX-1 (i.e., 98%).

**Table 1**

| Inhibitory activity of 100 µM compound (%) | | |
|---|---|---|
| | COX-1 | COX-2 |
| Compound 1 | 98 | 24 |
| Compound 2 | 32 | 27 |
| Compound 3 | 63 | 30 |
| Compound 4 | 76 | 31 |
| Compound 5 | 71 | 14 |
| Compound 6 | 70 | 41 |
| Compound 10 | 23 | 15 |
| Compound 11 | 23 | 20 |
| Compound 14 | 65 | 15 |

### In vitro test (2)

Inhibitory activities (IC₅₀) against cyclooxygenase-1 (COX-1) and cyclooxygenase 2 (COX-2) of compounds 1 and 14, which had exhibited particularly high COX-1-selective inhibitory activity as seen in Table 1, were determined with the use of the Cayman Cyclooxygenase Inhibitor Screening Assay Kit (760111). IC₅₀ values of compound 1 for COXs were 1 µM (COX-1) and >100 µM (COX-2). IC₅₀ values of compound 14 for COXs were 30 µM (COX-1) and >100 µM (COX-2). Also, IC₅₀ values of aspirin for COXs were 100 µM (COX-1) and >100 µM (COX-2).

**Table 2**

| | IC₅₀ (µM) | |
|---|---|---|
| | COX-1 | COX-2 |
| Aspirin | 100 | > 100 |
| Compound 1 | 1 | > 100 |
| Compound 14 | 30 | > 100 |

### In vivo test

### 1) Acetic acid writhing

Analgesic testing was carried out via common mice-based acetic acid writhing test. Compounds were administered orally in amounts of 30 mg/kg to groups each consisting of 10 or 11 mice, and a substance that applies pain stimuli, i.e., a 0.7% acetic acid solution, was intraperitoneally administered 30 minutes thereafter (0.1 ml/10g). The counting of mice-specific writhing responses was initiated 10 minutes after the administration of the acetic acid solution, and the counting was continued for 10 minutes. This test utilizes the fact that a distinct symptom of agony, that is a writhing response, occurs upon administration of a stimulatory substance, such as an acetic acid solution, to a mouse. As a result, the number of writhing responses was found to be 22 on average when compound 1 was not administered. When compound 1 was administered in amounts of 30 mg/kg, however, the number of writhing responses was 5 on average. When compound 1 was administered in amounts of 10 mg/kg, the number of writhing responses was 10 on average. When compound 14 was administered in amounts of 30 mg/kg, the number of writhing responses was 6 on average. This indicates that administration of compound 1 or 14 significantly suppresses the number of writhing responses, which in turn verifies the analgesic effects of compounds 1 and 14.

**Table 3**

| | Number of writhing responses |
|---|---|
| No compound | 22 |
| Aspirin (30 mg/kg) | 15 |
| Aspirin (10 mg/kg) | 17 |
| Compound 1 (30 mg/kg) | 5 |
| Compound 1 (10 mg/kg) | 10 |
| Compound 14 (30 mg/kg) | 6 |

### 2) Antiinflammatory effects (carrageenin edema test)

Antiinflammatory testing was carried out via a rat-based carrageenin edema test. Compounds were orally administered in amounts of 30 mg/kg to groups each consisting of 5 to 8 mice, and an edema-inducing substance (a 1% carrageenin solution) was administered hypodermically to footpads of hind paws 1 hour later (0.1 ml/mouse). Edema thickness in the footpads of hind paws was measured 1, 2, and 3 hours thereafter. As a result, the inhibitory activity of aspirin against edema 3 hours after the administration of the carrageenin was found to be 16%, and that of compound 1 was found to be 31 %.

**Table 4**

| | Inhibitory activity against edema (%) |
|---|---|
| No compound | 0 |
| Aspirin (30 mg/kg) | 16 |
| Compound 1 (30 mg/kg) | 31 |

### 3) Gastric ulcer formation

Compound 1 was orally administered in amounts of 30 mg/kg to groups each consisting of 3 to 5 mice, mice were sacrificed with the use of ether 5 hours later, and the stomachs were extracted. The extracted stomachs were opened, each entire stomach was photographed using a stereoscopic microscope, and the lengths of the gastric ulcers were determined. As a result, aspirin was found to give rise to significant gastric ulcers; however, gastric ulcer formation was not observed upon administration of compound 1 or 14.

**Table 5**

| | Total length of gastric ulcer (mm) |
|---|---|
| Aspirin (30 mg/kg) | 1.61 |
| Compound 1 (30 mg/kg) | 0.09 |
| Compound 14 (30 mg/kg) | 0.01 |

### 4) Toxicity test

Compound 1 was intraperitoneally administered in amounts of 1 g/kg to a group consisting of 3 mice, and the mice were observed for 10 days. This group of mice did not die upon administration of compound 1, and rapid decrease of body weight, changes in coats, and caudal necrosis were not observed, in comparison with the case of a group of mice to which no compound had been administered.

Compound 14 was intraperitoneally administered in amounts of 300 mg/kg to a group consisting of 5 mice, and the mice were observed for 14 days. This group of mice did not die upon administration of compound 14, and rapid decrease of body weight, changes in coats, and caudal necrosis was not observed, in comparison with the case of a group of mice to which no compound had been administered.

All publications, patents, and patent applications cited herein are incorporated herein by reference in their entirety.

## Claims

1. A compound represented by the following formula or a salt thereof: 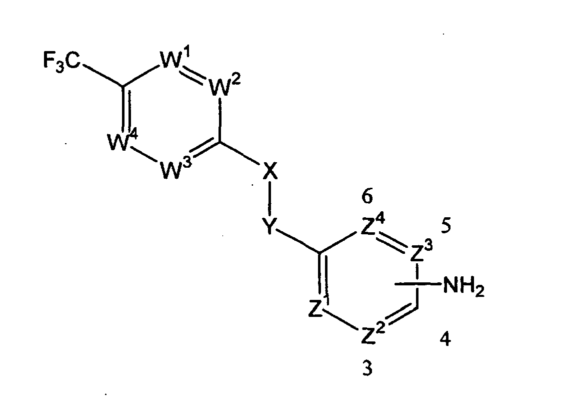 wherein either X or Y represents CO and the other represents NH; W¹, W², W³, and W⁴ each independently represent CH or N; Z¹, Z², Z³, and Z⁴ each independently represent CH or N (provided that Z², Z³, or Z⁴ to which an amino group binds is CH); and an amino group is located at position 3, 4, 5, or 6.

2. The compound according to claim 1 or a salt thereof, wherein X represents CO; Y represents NH; Z¹ represents N; Z², Z³, and Z⁴ each independently represent CH; W¹, W², W³, and W⁴ each independently represent CH; and an amino group is located at position 4.

3. The compound according to claim 1 or a salt thereof, wherein X represents CO; Y represents NH; Z¹ represents N; Z², Z³, and Z⁴ each independently represent CH; W¹, W², W³, and W⁴ each independently represent CH; and an amino group is located at position 6.

4. The compound according to claim 1 or a salt thereof, wherein X represents CO; Y represents NH; Z¹, Z², Z³, and Z⁴ each independently represent CH; W¹, W², W³, and W⁴ each independently represent CH; and an amino group is located at position 4.

5. The compound according to claim 1 or a salt thereof, wherein X represents CO; Y represents NH; Z¹, Z², Z³, and Z⁴ each independently represent CH; W¹, W², W³, and W⁴ each independently represent CH; and an amino group is located at position 5.

6. The compound according to claim 1 or a salt thereof, wherein X represents NH; Y represents CO; Z¹, Z², Z³, and Z⁴ each independently represent CH; W¹, W², W³, and W⁴ each independently represent CH; and an amino group is located at position 4.

7. The compound according to claim 1 or a salt thereof, wherein X represents NH; Y represents CO; Z¹, Z², Z³, and Z⁴ each independently represent CH; W¹, W², W³, and W⁴ each independently represent CH; and an amino group is located at position 5.

8. The compound according to claim 1 or a salt thereof, wherein X represents CO; Y represents NH; Z¹ and Z⁴ each independently represent N; Z² and Z⁴ each independently represent CH; W¹, W², W³, and W⁴ each independently represent CH; and an amino group is located at position 4.

9. The compound according to claim 1 or a salt thereof, wherein X represents CO; Y represents NH; Z¹ represents N; Z², Z³, and Z⁴ each independently represent CH; W¹ represents N; W², W³, and W⁴ each independently represent CH; and an amino group is located at position 4.

10. The compound according to claim 1 or a salt thereof, wherein X represents CO; Y represents NH; Z¹ represents N; Z¹, Z³, and Z⁴ each independently represent CH; W¹ and W² represents N; W³ and W⁴ each independently represent CH; and an amino group is located at position 4.

11. The compound according to claim 1 or a salt thereof, wherein X represents NH; Y represents CO; Z¹ represents N; Z², Z³, and Z⁴ each independently represent CH; W¹, W², W³, and W⁴ each independently represent CH; and an amino group is located at position 4.

12. The compound according to claim 1 or a salt thereof, wherein X represents NH; Y represents CO; Z¹ represents N; Z², Z³, and Z⁴ each independently represent CH; W¹ represents N; W², W³, and W⁴ each independently represent CH; and an amino group is located at position 4.

13. The compound according to claim 1 or a salt thereof, wherein X represents NH; Y represents CO; Z¹ represents N; Z², Z³, and Z⁴ each independently represent CH; W² represents N; W¹, W³, and W⁴ each independently represent CH; and an amino group is located at position 4.

14. The compound according to claim 1 or a salt thereof, wherein X represents CO; Y represents NH; Z¹ represents N; Z², Z³, and Z⁴ each independently represent CH; W¹, W², W³, and W⁴ each independently represent CH; and an amino group is located at position 5.

15. The compound according to claim 1 or a salt thereof, wherein X represents NH; Y represents CO; Z¹, Z², Z³, and Z⁴ each independently represent CH; W² represents N; W¹, W³, and W⁴ each independently represent CH; and an amino group is located at position 4.

16. An analgesic agent comprising, as an active ingredient, the compound according to any one of claims 1 to 15 or a pharmaceutically acceptable salt thereof.

17. An antiinflammatory agent comprising, as an active ingredient, the compound according to any one of claims 1 to 15 or a pharmaceutically acceptable salt thereof.

18. An antitumor agent comprising, as an active ingredient, the compound according to any one of claims 1 to 15 or a pharmaceutically acceptable salt thereof.

19. An antiplatelet aggregation agent comprising, as an active ingredient, the compound according to any one of claims 1 to 15 or a pharmaceutically acceptable salt thereof.

20. A cyclooxygenase-1-selective inhibitor comprising, as an active ingredient, the compound according to any one of claims 1 to 15 or a pharmaceutically acceptable salt thereof.
